(19) Europäisches Patentamt
European Patent Office
Office européen des brevets

(11) **EP 4 349 415 A1**

(12) **EUROPEAN PATENT APPLICATION**
published in accordance with Art. 153(4) EPC

(43) Date of publication:
**10.04.2024 Bulletin 2024/15**

(21) Application number: **22815932.3**

(22) Date of filing: **25.05.2022**

(51) International Patent Classification (IPC):
*A61Q 1/00* (2006.01)    *A61K 8/06* (2006.01)
*A61K 8/19* (2006.01)    *A61K 8/37* (2006.01)
*A61K 8/891* (2006.01)    *A61K 8/894* (2006.01)

(52) Cooperative Patent Classification (CPC):
**A61K 8/06; A61K 8/19; A61K 8/37; A61K 8/891;
A61K 8/894; A61Q 1/00; A61Q 1/02**

(86) International application number:
**PCT/JP2022/021368**

(87) International publication number:
**WO 2022/255182 (08.12.2022 Gazette 2022/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.06.2021 JP 2021092878**

(71) Applicant: **Kao Corporation
Chuo-ku
Tokyo 103-8210 (JP)**

(72) Inventors:
- **FUJITANI, Tomomi
  Odawara-shi, Kanagawa 250-0002 (JP)**
- **KITAMURA, Toshihiko
  Odawara-shi, Kanagawa 250-0002 (JP)**

(74) Representative: **Hoffmann Eitle
Patent- und Rechtsanwälte PartmbB
Arabellastraße 30
81925 München (DE)**

(54) **WATER-IN-OIL EMULSION COMPOSITION**

(57) A water-in-oil emulsion composition is provided to comprise components (A), (B), (C) and (D): (A) a cationically modified clay mineral substituted with a quaternary ammonium ion; (B) a phenyl-modified silicone in liquid form at 25°C; (C) a sorbitan fatty acid ester, wherein a content of the component (C) is from 0.00005 to 0.3 mass%; and (D) a polyether-modified silicone.

**EP 4 349 415 A1**

**Description**

Field of the Invention

The present invention relates to a water-in-oil emulsion composition.

Background of the Invention

Conventionally, water-in-oil emulsion cosmetics excellent in long-lasting makeup and use impression have been studied.
For example, PTL1 discloses a water-in-oil emulsion cosmetic containing a cationically modified clay mineral, a hydrophobically treated color pigment, a phenyl-modified silicone, and a volatile oil, and it is also disclosed that the cosmetic adheres well to the skin, spreads lightly and easily, gives the skin a thin film feel and a glow, and results in a uniform and beautiful finish on the skin surface.
PTL1: JP-A-2018-70516

Summary of the Invention

The present invention relates to a water-in-oil emulsion composition comprising following components (A), (B), (C) and (D):

  (A) a cationically modified clay mineral substituted with a quaternary ammonium ion;
  (B) a phenyl-modified silicone in liquid form at 25°C;
  (C) a sorbitan fatty acid ester, wherein a content of the component (C) is from 0.00005 to 0.3 mass%; and
  (D) a polyether-modified silicone.

Detailed Description of the Invention

Conventional cosmetics have a problem of poor viscosity stability when stored for a long period of time. In particular, cosmetics with relatively high viscosity have the following problem, for example: in the case where a pressure is applied to the cosmetic using a filling machine to fill the cosmetic into a container, the viscosity of the cosmetic is hardly recovered to that before the pressure application.
Further, the conventional cosmetics have not been sufficiently satisfied in terms of the spreadability when applied, the feel of adhesion to the skin, and the covering ability of the finish.
The present inventors found that by using a very small amount of a sorbitan fatty acid ester together with a cationically modified clay mineral, a phenyl-modified silicone and a polyether-modified silicone, a water-in-oil emulsion composition can be obtained that has good viscosity stability to easily recover its viscosity even when the viscosity is lowered by pressure application to the cosmetic, and that is excellent in the spreadability when applied, the feel of adhesion to the skin, and the covering ability of the finish.
The water-in-oil emulsion composition of the present invention is excellent in viscosity stability even after long-term storage.
In the present invention, excellent viscosity stability even after long-term storage means that even when the viscosity of the water-in-oil emulsion composition is lowered by pressure application to the composition by a filling machine or the like, the viscosity is easily recovered to that before the pressure application.
Further, the water-in-oil emulsion composition of the present invention is also excellent in the spreadability when applied, the feel of adhesion to the skin, and the covering ability of the finish.
As the cationically modified clay mineral of the component (A) used in the present invention, clay minerals substituted with a quaternary ammonium ion and used for ordinary cosmetics may be used without limitations. Preferred examples thereof include cationically modified clay minerals obtained by substituting layered clay minerals such as bentonite, laponite, hectorite, montmorillonite and aluminum magnesium silicate with a quaternary ammonium salt-type cationic surfactant.
Here, the quaternary ammonium salt-type cationic surfactant is represented by the following formula (1):

$$[R^1R^2R^3R^4N]^+ \, X^- \qquad (1)$$

wherein $R^1$ represents an alkyl group having from 10 to 22 carbon atoms or a benzyl group, $R^2$ represents a methyl group or an alkyl group having from 10 to 22 carbon atoms, and $R^3$ and $R^4$ each represent an alkyl group or a hydroxyalkyl group having from 1 to 3 carbon atoms, and X represents a halogen atom or a methylsulfate residue.

Specific examples thereof include dodecyltrimethylammonium chloride, myristyltrimethylammonium chloride, cetyltrimethylammonium chloride, stearyltrimethylammonium chloride, behenyltrimethylammonium chloride, myristyldimethylethylammonium chloride, cetyldimethylethylammonium chloride, stearyldimethylethylammonium chloride, behenyldimethylethylammonium chloride, myristyldiethylmethylammonium chloride, cetyldiethylmethylammonium chloride, stearyldiethylmethylammonium chloride, behenyldiethylmethylammonium chloride, benzyldimethylmyristylammonium chloride, benzyldimethylcetylammonium chloride, benzyldimethylstearylammonium chloride, benzyldimethylbehenylammonium chloride, benzylmethylethylcetylammonium chloride, benzylmethylethylstearylammonium chloride, distearyldimethylammonium chloride, dibehenyldihydroxyethylammonium chloride, bromide compounds instead of each of the chloride compounds, and dipalmitylpropylethylammonium methylsulfate.

Among them, the component (A) preferably includes one or more selected from the group consisting of benzyldimethylstearylammonium chloride and dimethyldistearylammonium chloride, and dimethyldistearylammonium chloride is more preferred.

The cationically modified clay mineral obtained by substituting a layered clay mineral with a quaternary ammonium salt-type cationic surfactant preferably includes one or more selected from the group consisting of dimethyldistearylammonium hectorite, dimethyldistearylammonium bentonite, and benzyldimethylstearylammonium hectorite, more preferably includes one or more selected from the group consisting of dimethyldistearylammonium hectorite and benzyldimethylstearylammonium hectorite, and further more preferably includes dimethyldistearylammonium hectorite. Examples of commercially available products include Benton 38, Benton 38VCG, and Benton 27 (manufactured by Elementis Japan).

Organic substance-modified clay minerals may be used in the form of a dispersion diluted with a solvent, in view of excellent workability and the thickening effect on oils.

Specifically, it is preferred to use a premix gel including an organic substance-modified clay mineral dispersed in a solvent in advance. The solvent is not limited as long as it can be thickened by an organic substance-modified clay mineral. In view of the thickening effect on oils, the solvent preferably includes one or more selected from the group consisting of octyldodecanol, a mineral oil, a volatile silicone oil, glyceryl tri(caprylate/caprate), and an alkyl benzoate (from C12 to C15), and more preferably includes one or more selected from the group consisting of glyceryl tri(caprylate/caprate) and alkyl benzoate (from C12 to C15).

The content of the organic substance-modified clay mineral in the premix gel is preferably from 5 to 25 mass%, more preferably from 10 to 20 mass%, and further more preferably from 10 to 18 mass%, in view of the improved workability, the thickening effect on oils, and suppression of the oil separation of the thickened oily gel itself.

As the premix gel, commercially available products may be used, such as Benton gel EUGV and Benton gel MIOV, which contain 10 mass% of a cationically modified clay mineral, Benton gel VS-5 PCV, which contains 18 mass% of a cationically modified clay mineral, Benton gel PTM, which contains 15 mass% of a cationically modified clay mineral (manufactured by Elementis Japan), and BENTONE GEL GTCC V and BENTONE GEL TN V, which contain 12.5 mass% of a cationically modified clay mineral (manufactured by Elementis Specialties).

One or more cationically modified clay minerals may be used for the component (A). In view of improving the viscosity stability, the spreadability when applied, the feel of adhesion to the skin, the covering ability of the finish, and the clean finish feel of the entire face, the content thereof in the whole composition is preferably 0.01 mass% or more, more preferably 0.1 mass% or more, further more preferably 0.5 mass% or more, even more preferably 0.8 mass% or more, particularly preferably 1.4 mass% or more, and preferably 10 mass% or less, more preferably 5 mass% or less, further more preferably 3 mass% or less, even more preferably 2.5 mass% or less, and particularly preferably 1.8 mass% or less. Also, the content of component (A) in the whole composition is preferably from 0.01 to 10 mass%, more preferably from 0.1 to 5 mass%, further more preferably from 0.5 to 3 mass%, even more preferably from 0.8 to 2.5 mass%, and particularly preferably from 1.4 to 1.8 mass%.

The component (B) is a phenyl-modified silicone in liquid form at 25°C. The term "liquid form" means that the viscosity at 25°C is 10 000 mPa·s or less.

The viscosity of the component (B) at 25°C is preferably 4 000 mPa·s or less, more preferably 600 mPa·s or less, further more preferably 200 mPa·s or less, and even more preferably 50 mPa·s or less.

Here, the viscosity is measured under the following conditions.

Viscosity measurement conditions: B-type viscometer (Model TVB-10, manufactured by Toki Sangyo Co., Ltd.), rotor No. 4, 60 rpm, 60 seconds.

Examples of the phenyl-modified silicones in liquid form at 25°C include phenyl trimethicone, diphenylsiloxy phenyl trimethicone, diphenyl dimethicone, trimethylsiloxy phenyl dimethicone, and trimethyl pentaphenyl trisiloxane.

Among them, in view of improving the viscosity stability, spreadability when applied, the feel of adhesion to the skin, the covering ability of the finish, and the feel of clean finish of the entire face, the component (B) preferably includes one or more selected from the group consisting of phenyl trimethicone and diphenylsiloxy phenyl trimethicone, and more preferably includes diphenylsiloxy phenyl trimethicone.

As the component (B), commercially available products may be used, such as SH556 (manufactured by Dow Corning Toray) and DOWSIL SH 556 FLUID (manufactured by Dow Toray) as phenyl trimethicone; KF-56A (manufactured by Shin-Etsu Chemical Co., Ltd.) and DOWSIL FZ-209 (manufactured by Dow Toray) as diphenylsiloxy phenyl trimethicone; KF-50 (manufactured by Shin-Etsu Chemical Co., Ltd.), KF-53 (manufactured by Shin-Etsu Chemical Co., Ltd.), KF-54 (manufactured by Shin-Etsu Chemical Co., Ltd.) as diphenyl dimethicone; PDM-1000 (manufactured by Wacker Asahikasei Silicone Co., Ltd.) as trimethylsiloxy phenyl dimethicone; and PH-1555 HRI C. F. (manufactured by Dow Corning Toray) as trimethyl pentaphenyl trisiloxane.

As the component (B), one or more kinds may be used. In view of improving the viscosity stability, the spreadability when applied, the feel of adhesion to the skin, the covering ability of the finish, and the clean finish feel of the entire face, the content thereof in the whole composition is preferably 1 mass% or more, more preferably 2 mass% or more, further more preferably 3 mass% or more, even more preferably 3.5 mass% or more, particularly preferably 5 mass% or more, and preferably 25 mass% or less, more preferably 20 mass% or less, further more preferably 15 mass% or less, even more preferably 10 mass% or less, and particularly preferably 7 mass% or less. Also, the content of component (B) in the whole composition is preferably from 1 to 25 mass%, more preferably from 2 to 20 mass%, further more preferably from 3 to 15 mass%, even more preferably from 3.5 to 10 mass%, and particularly preferably from 5 to 7 mass%.

In the present invention, in view of improving the viscosity stability, the spreadability when applied, the feel of adhesion to the skin, the covering ability of the finish, and the clean finish feel of the entire face, the mass ratio of the component (A) to the component (B), (A)/(B), is preferably 0.05 or more, more preferably 0.1 or more, further more preferably 0.12 or more, even more preferably 0.15 or more, more preferably 0.2 or more, even more preferably 0.23 or more, and preferably 1 or less, more preferably 0.5 or less, further more preferably 0.45 or less, even more preferably 0.40 or less, more preferably 0.36 or less, and even more preferably 0.3 or less. Also, the mass ratio of the component (A) to the component (B), (A)/(B), is preferably from 0.05 to 1, more preferably from 0.1 to 0.5, further more preferably from 0.12 to 0.45, even more preferably from 0.15 to 0.40, more preferably 0.2 to 0.36, and further more preferably from 0.23 to 0.3.

The component (C) used in the present invention is a sorbitan fatty acid ester.

In view of improving the viscosity stability, the spreadability when applied, the feel of adhesion to the skin, the covering ability of the finish, and the clean finish feel of the entire face, the sorbitan fatty acid ester has an HLB of preferably 1 or more, more preferably 2 or more, further more preferably 3 or more, and preferably 10 or less, more preferably 7 or less, and further more preferably 6 or less. Also, sorbitan fatty acid ester of the component (C) has an HLB of preferably from 1 to 10, more preferably from 2 to 7, further more preferably from 3 to 6.

In the present invention, HLB (Hydrophilic-Lipophilic Balance) indicates the molecular weight of the hydrophilic group moiety in the total molecular weight of a surfactant, and is determined by the Griffin equation. In the case of a mixed surfactant including two or more nonionic surfactants, the HLB thereof is obtained in the following manner. The HLB of a mixed surfactant is obtained by addition-averaging the HLB values of the respective nonionic surfactants on the blending ratio basis.

$$\text{Mixed HLB} = \Sigma(\text{HLBx} \times \text{Wx}) / \Sigma \text{Wx}$$

wherein HLBx represents the HLB value of a nonionic surfactant X, and Wx represents the mass (g) of the nonionic surfactant X having a value of HLBx.

Examples of the sorbitan fatty acid esters include sorbitan monoisostearate, sorbitan monooleate, sorbitan sesquistearate, and sorbitan sesquioleate. In view of improving the viscosity stability, the spreadability when applied, the feel of adhesion to the skin, the covering ability of the finish, and the clean finish feel of the entire face, the component (C) preferably includes one or more selected from the group consisting of sorbitan monoisostearate, sorbitan monooleate, and sorbitan sesquistearate, more preferably includes one or more selected from the group consisting of sorbitan monoisostearate and sorbitan sesquistearate, and further more preferably includes sorbitan monoisostearate.

As the sorbitan fatty acid ester of the component (C), for example, commercially available products may be used, such as Span 120 (HLB: 4.7) (manufactured by Croda Japan K.K.) as sorbitan monoisostearate, NIKKOL SS-15V (HLB: 4.2) as sorbitan sesquistearate, and NIKKOL SO-10V (HLB: 4.3) (manufactured by Nikko Chemicals Co., Ltd.) as sorbitan monooleate.

In view of improving the viscosity stability, the spreadability when applied, the feel of adhesion to the skin, the covering ability of the finish, and the clean finish feel of the entire face, the content of the component (C) in the whole composition is 0.00005 mass% or more, preferably 0.00007 mass% or more, more preferably 0.00008 mass% or more, further more preferably 0.0001 mass% or more, even more preferably 0.0005 mass% or more, and 0.3 mass% or less, preferably 0.1 mass% or less, more preferably 0.05 mass% or less, further more preferably 0.005 mass% or less. Also, the content of the component (C) in the whole composition is from 0.00005 to 0.3 mass%, preferably from 0.00007 to 0.1 mass%, more preferably from 0.00008 to 0.05 mass%, further more preferably from 0.0001 to 0.05 mass%, and even more preferably from 0.0005 to 0.005 mass%.

Also, the content of the component (C) in the whole composition is preferably from 0.0001 to 0.3 mass%.

In the present invention, in view of improving the viscosity stability, the spreadability when applied, the feel of adhesion to the skin, the covering ability of the finish, and the clean finish feel of the entire face, the mass ratio of the component (B) to the component (C), (B)/(C), is preferably 20 or more, more preferably 60 or more, further more preferably 150 or more, even more preferably 200 or more, even more preferably 2 000 or more, and preferably 80 000 or less, more preferably 60 000 or less, further more preferably 30 000 or less, and even more preferably 7 000 or less. Also, the mass ratio of the component (B) to the component (C), (B)/(C), is preferably from 20 to 80 000, more preferably from 60 to 60 000, further more preferably from 150 to 30 000, even more preferably from 200 to 30 000, even more preferably from 2 000 to 7 000.

Examples of the polyether-modified silicone of the component (D) include ones used in ordinary cosmetics, such as a dimethylpolysiloxane having a polyoxyethylene group or a polyoxypropylene group, and a dimethylpolysiloxane to which polyethylene glycol is added. The dimethylpolysiloxane may be partially modified with fluorine.

In view of improving the viscosity stability, the spreadability when applied, the feel of adhesion to the skin, the covering ability of the finish, and the clean finish feel of the entire face, a dimethylpolysiloxane having a polyoxyethylene group or a polyoxypropylene group is preferred as the polyether-modified silicones, and a dimethylpolysiloxane having at least a polyoxyethylene group is more preferred.

It is preferable that the polyether-modified silicone contain no crosslinked one.

In view of improving the viscosity stability, the spreadability when applied, the feel of adhesion to the skin, the covering ability of the finish, and the clean finish feel of the entire face, the polyether-modified silicone has an HLB of preferably 1 or more, more preferably 3 or more, and preferably 12 or less, more preferably 10 or less, further more preferably 8 or less, even more preferably 6 or less.

Here, HLB is calculated in the same manner as for the component (C).

Examples of the polyether-modified silicones include cetyl PEG/PPG-10/1 dimethicone, PEG/PPG-19/19 dimethicone, PEG10-dimethicone, PEG-9 polydimethylsiloxyethyl dimethicone, and lauryl PEG-9 polydimethylsiloxyethyl dimethicone. Among these, in view of improving the viscosity stability, the spreadability when applied, the feel of adhesion to the skin, the covering ability of the finish, and the clean finish feel of the entire face, the component (D) preferably includes one or more selected from the group consisting of cetyl PEG/PPG-10/1 dimethicone, PEG/PPG-19/19 dimethicone, and PEG10-dimethicone, more preferably includes one or more selected from the group consisting of cetyl PEG/PPG-10/1 dimethicone and PEG/PPG-19/19 dimethicone, and further more preferably includes cetyl PEG/PPG-10/1 dimethicone.

As the component (D), one or more kinds may be used. In view of improving the viscosity stability, the spreadability when applied, the feel of adhesion to the skin, the covering ability of the finish, and the clean finish feel of the entire face, the content of the components (D) in the whole composition is preferably 0.1 mass% or more, more preferably 0.5 mass% or more, further more preferably 1 mass% or more, and preferably 10 mass% or less, more preferably 5 mass% or less, further more preferably 3 mass% or less. Also, the content of the component (D) in the whole composition is preferably from 0.1 to 10 mass%, more preferably from 0.5 to 5 mass%, further more preferably from 1 to 3 mass%.

The water-in-oil emulsion composition of the present invention may further contain an ester oil (E) in liquid form at 25°C to improve the viscosity stability, the spreadability when applied, and the feel of adhesion to the skin. Here, the term "liquid form" means that the viscosity at 25°C is 10 000 mPa·s or less, as with the component (B). The viscosity of the component (E) is measured in the same manner as for the component (B).

As the ester oil of the component (E), those used in ordinary cosmetics may be used. Examples thereof include monoester oils such as ethylhexyl para-methoxycinnamate, cetyl ethylhexanoate, octyldodecyl myristate, isotridecyl isononanoate, and isononyl isononanoate; diester oils such as neopentyl glycol dicaprate, propylene glycol dicaprate, and diisostearyl malate; triester oils such as glyceryl tri(2-ethylhexanoate), glyceryl tri(caprylate/caprate); and tetraester oils such as dipentaerythrityl tetraisostearate.

Among these, in view of improving the viscosity stability, the spreadability when applied, and the feel of adhesion to the skin, the component (D) preferably includes one or more selected from the group consisting of a monoester, a diester, and a triester, and more preferably includes one or more selected from the group consisting of a monoester and a diester. Also, it is more preferable to use a monoester and a diester in combination.

As the component (E), the ester oils may be used singly or in combinations of two or more thereof. In view of improving the viscosity stability, the spreadability when applied, and the feel of adhesion to the skin, the content of the component (E) in the whole composition is preferably 1 mass% or more, more preferably 2 mass% or more, further more preferably 3 mass% or more, and preferably 20 mass% or less, more preferably 15 mass% or less, further more preferably 10 mass% or less. Also, the content of the component (E) in the whole composition is preferably from 1 to 20 mass%, more preferably from 2 to 15 mass%, further more preferably from 3 to 10 mass%.

The water-in-oil emulsion composition of the present invention may further contain an oily gelling agent other than the component (A) as a component (F) to improve the viscosity stability, the spreadability when applied, the feel of adhesion to the skin, the covering ability of the finish, and the clean finish feel of the entire face.

The oily gelling agent may be any one used in ordinary cosmetics for thickening the oil phase.

Examples of the oily gelling agent of the component (F) include a crosslinked organopolysiloxane, a dextrin fatty acid ester, a metal soap, a sucrose fatty acid ester, and oily component (wax) in solid form at 25°C.

Examples of the crosslinked organopolysiloxane include a crosslinked dimethylpolysiloxane, a crosslinked alkylpolysiloxane, and a crosslinked polyether-modified silicone.

The crosslinked dimethylpolysiloxane is a polymer having a crosslinked structure with a siloxane skeleton three-dimensionally crosslinked, and the crosslinked alkylpolysiloxane is a crosslinked dimethylpolysiloxane further having an alkyl group having from 6 to 20 carbon atoms.

Examples of the crosslinked dimethylpolysiloxane include a (dimethicone/vinyl dimethicone) crosspolymer, and examples of the crosslinked alkylpolysiloxane include a (dimethicone/phenyl dimethicone) crosspolymer, a (vinyl dimethicone/lauryl dimethicone) crosspolymer, an alkyl(from C30 to C45)cetearyl dimethicone crosspolymer, and a cetearyl dimethicone crosspolymer.

These may be used as they are in solid form, or may be used after uniformly mixing with a liquid oil. In particular, in view of dispersibility into a cosmetic and workability, it is preferred to use in the form of a dispersion diluted with a liquid oil. It is preferred to use one or more selected from the group consisting of a silicone oil, a hydrocarbon oil, and an ester oil; it is more preferred to use one or more selected from the group consisting of a silicone oil and a hydrocarbon oil; it is further more preferred to use one or more selected from the group consisting of dimethylpolysiloxane, methyltrimethicone, decamethyl cyclopentasiloxane, a liquid paraffin, and a light isoparaffin; and it is even more preferred to use one or more selected from the group consisting of dimethylpolysiloxane and methyltrimethicone.

As the (dimethicone/vinyl dimethicone) crosspolymer, a commercially available product may be used, such as KSG-15, which is a mixture with decamethyl cyclopentasiloxane, KSG-16, which is a mixture with low-viscosity dimethylpolysiloxane (manufactured by Shin-Etsu Chemical Co., Ltd.), and TOREFIL (35%) C/TMF (manufactured by Daito Kasei Kogyo Co., Ltd.), which is a mixture with methyltrimethicone.

As the (dimethicone/phenyldimethicone) crosspolymer, a commercially available product may be used, such as KSG-18 (manufactured by Shin-Etsu Chemical Co., Ltd.), which is a mixture with methylphenylpolysiloxane.

Also, as the (vinyl dimethicone/lauryl dimethicone) crosspolymer, a commercially available product may be used, such as KSG-41, which is a mixture with liquid paraffin, KSG-42, which is a mixture with light isoparaffin, and KSG-43, which is a mixture with glyceryl tri-2-ethylhexanoate, and KSG-44, which is a mixture with squalane (manufactured by Shin-Etsu Chemical Co., Ltd.).

Furthermore, as the alkyl (from C30 to C45) cetearyl dimethicone crosspolymer, a commercially available product may be used, such as Velvesil 125, which is a mixture with cyclopentasiloxane (manufactured by Momentive Performance Materials).

As the cetearyl dimethicone crosspolymer, a commercially available product may be used, such as Velvesil DM, which is a mixture of dimethicone(manufactured by Momentive Performance Materials).

Examples of the crosslinked polyether-modified silicone include a (dimethicone/(PEG-10/15)) crosspolymer, a (PEG-15/lauryl dimethicone) crosspolymer, and a (PEG-10/lauryl dimethicone) crosspolymer.

In view of dispersibility into a cosmetic and workability, it is preferred to use the crosslinked polyether-modified silicone in the form of a dispersion diluted with a liquid oil. A silicone oil, a hydrocarbon oil, or an ester oil is more preferably used, and a silicone oil or a hydrocarbon oil is further more preferably used.

Furthermore, a crosslinked polyether-modified silicone diluted or dispersed in a volatile hydrocarbon oil are preferred, and isododecane is more preferred as the volatile hydrocarbon oil.

As the crosslinked polyether-modified silicones, a commercially available product may be used, such as KSG-210, KSG-240 ((dimethicone/(PEG-10/15)) crosspolymer); KSG-310, KSG-320, KSG-330 ((PEG-15/lauryl dimethicone) crosspolymer); KSG-340 ((PEG-10/lauryl dimethicone) crosspolymer and (PEG-15/lauryl dimethicone) crosspolymer) (manufactured by Shin-Etsu Chemical Co., Ltd.).

It is preferable that the crosslinked organopolysiloxane contains one or more selected from the group consisting of a crosslinked alkylpolysiloxane and a crosslinked dimethylpolysiloxane. A (dimethicone/vinyl dimethicone) crosspolymer, a (dimethicone/phenyl dimethicone) crosspolymer, and a (vinyl dimethicone/lauryl dimethicone) crosspolymer are more preferred, and a (dimethicone/vinyl dimethicone) crosspolymer is further more preferred.

The dextrin fatty acid ester is an ester of a fatty acid and dextrin, and the fatty acid preferably has from 8 to 24 carbon atoms, more preferably from 14 to 18 carbon atoms. The fatty acid is a linear or branched saturated or unsaturated fatty acid. The average polymerization degree is preferably from 10 to 50, more preferably from 20 to 30.

Specific examples thereof include dextrin palmitate, dextrin palmitate/2-ethylhexanoate, dextrin stearate, dextrin palmitate/stearate, dextrin oleate, dextrin isopalmitate, dextrin isostearate, and dextrin myristate.

As the dextrin fatty acid ester, dextrin palmitate and dextrin palmitate/2-ethylhexanoate are preferred, and dextrin palmitate is more preferred.

Examples of the commercially available products thereof include dextrin palmitate (Rheopearl KL2, Rheopearl KS2, and Rheopearl TL2), dextrin palmitate/2-ethylhexanoate (Rheopearl TT2), and dextrin myristate (Rheopearl MKL2) manufactured by Chiba Flour Milling Co., Ltd.

Examples of oily components in solid form at 25°C include a wax such as carnauba wax (melting point: from 80 to 86°C), beeswax (melting point: 64°C), candelilla wax (melting point: from 68 to 72°C), and rice bran wax (melting point: from 70 to 83°C), jojoba wax (melting point: 55°C), Japan wax (melting point: 55°C), jojoba fat (melting point: from 46 to 54°C), and lanolin (melting point: from 37 to 43°C); an ester oil such as hydrogenated jojoba oil (melting point: 68°C), isostearic acid hydrogenated castor oil (melting point: 45°C), hydrogenated castor oil (melting point: 84°C), hydrogenated palm oil (melting point: 65°C), hydrogenated coconut oil (melting point: 70°C), cetyl palmitate (melting point: 50°C), glyceryl behenate/eicosadioate (melting point: 66°C), glyceryl behenate, tristearin, tribehenin, shea butter (melting point: from 36 to 45°C), dimer dilinoleic acid (phytosteryl/isostearyl/cetyl/stearyl/behenyl) (melting point: 38°C), glyceryl tri(caprylate/caprate/myristate/stearate) (melting point: 40°C), phytosteryl oleate, and macadamia nut fatty acid phytosteryl; an ether oils such as batyl alcohol (melting point: from 60 to 70°C) and chimyl alcohol (melting point from 60.5 to 61.5°C); and a higher alcohol such as stearyl alcohol (melting point 58.0°C) and behenyl alcohol (melting point 70.5°C).

In view of improving the viscosity stability, the spreadability when applied, the feel of adhesion to the skin, the covering ability of the finish, and the clean finish feel of the entire face, the oily gelling agent of the component (F) preferably contains one or more selected from the group consisting of a crosslinked organopolysiloxane, a dextrin fatty acid ester, and an oily component in solid form at 25°C, and a crosslinked organopolysiloxane is more preferred. The oily gelling agent of the component (F) further more preferably contains one or more selected from the group consisting of a crosslinked dimethylpolysiloxane and a crosslinked alkylpolysiloxane, and even more preferably contains one or more selected from the group consisting of a (dimethicone/vinyl dimethicone) crosspolymer, a (dimethicone/phenyl dimethicone) crosspolymer, and a (vinyl dimethicone/lauryl dimethicone) crosspolymer. A (dimethicone/vinyl dimethicone) crosspolymer is particularly preferred.

As the component (F), the oily gelling agents other than the component (A) may be used singly or in combinations of two or more thereof. In view of improving the viscosity stability, the spreadability when applied, the feel of adhesion to the skin, the covering ability of the finish, and the clean finish feel of the entire face, the content thereof in the whole composition is preferably 0.1 mass% or more, more preferably 0.2 mass% or more, further more preferably 0.3 mass% or more, even more preferably 0.5 mass% or more, and preferably 20 mass% or less, more preferably 10 mass% or less, further more preferably 5 mass% or less, and even more preferably 2 mass% or less. Also, the content of the component (F) in the whole composition is preferably from 0.1 to 20 mass%, more preferably from 0.2 to 10 mass%, further more preferably from 0.3 to 5 mass%, and even more preferably from 0.5 to 2 mass%.

In view of improving the covering ability of the finish, and the clean makeup finish feel of the entire face, the water-in-oil emulsion composition of the present invention may further contain a color pigment (G).

Examples of the color pigment include ones used in ordinary cosmetics, such as a metal oxide such as titanium oxide, zinc oxide, cerium oxide, aluminum oxide, yellow iron oxide, black iron oxide, red iron oxide, Prussian blue, ultramarine blue, chromium oxide, and chromium hydroxide, a metal complex such as manganese violet and cobalt titanate, an inorganic pigment such as carbon black; a synthetic organic pigment, an organic coloring matter and a lake pigment thereof such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1 and Blue No. 404; and a natural organic coloring matter such as β- carotene, caramel and paprika pigment. Examples of the color pigment further include a composite of these color pigments and a mixed pigment obtained by diluting these color pigments with an extender pigment such as barium sulfate and talc.

In view of improving the viscosity stability, the spreadability when applied, the feel of adhesion to the skin, the covering ability of the finish, and the clean finish feel of the entire face, the color pigment contains preferably a metal oxide, more preferably one or more selected from the group consisting of titanium oxide, zinc oxide, yellow iron oxide, black iron oxide and red iron oxide, and further more preferably one or more selected from the group consisting of titanium oxide, yellow iron oxide, black iron oxide and red iron oxide.

These color pigments may be used as they are, or may be hydrophobized before use.

The hydrophobization treatment is not limited as long as it is a treatment applied to ordinary cosmetic powders, and dry processing or wet processing may be performed by using a surface treatment agent such as a silicone compound, a metal soap, an amino acid-based compound, lecithin, an oil, and an organic titanate.

Specific examples of the surface treatment agent include an alkyl alkoxysilane such as methyl trimethoxysilane, ethyl trimethoxysilane, hexyl trimethoxysilane, octyl trimethoxysilane, octyl triethoxysilane and triethoxy caprylylsilane, a silicone-based compound such as dimethylpolysiloxane, methyl hydrogen polysiloxane, a cyclic silicone, a crosslinked silicone, a silicone resin, and an acrylic-modified silicone; a metal soap such as aluminum stearate, aluminum myristate, zinc stearate, and magnesium stearate; an amino acid such as proline, hydroxyproline, alanine, glycine, sarcosine, glutamic acid, aspartic acid, lysine and a derivative thereof; an acylated amino acid such as stearoyl glutamic acid, lauroyl aspartic acid, lysine dilauroyl glutamate, and lauroyl lysine; lecithin and hydrogenated lecithin; an oil agent such as polyisobutylene, wax, oils and fats and a fatty acid; and an organic titanate such as isopropyl triisostearoyl titanate.

The acylated amino acid includes a salt of acylated amino acid. Examples of the salt of acylated amino acid include the salt of Na, Ca, Al, Mg, Zn, Zr or Ti. The salt of acylated amino acid preferably includes one or more selected from the group consisting of a Na salt and a Ca salt, and more preferably includes a Na salt. Further, in view of improving the viscosity stability, the spreadability when applied, the feel of adhesion to the skin, the covering ability of the finish, and the clean finish feel of the entire face, the salt of acylated amino acid preferably includes one or more selected from the group consisting of disodium stearoyl glutamate and sodium lauroyl aspartate, and more preferably includes disodium stearoyl glutamate.

In view of improving the viscosity stability, the spreadability when applied, the feel of adhesion to the skin, the covering ability of the finish, and the clean finish feel of the entire face, the surface treatment agent preferably includes one or more selected from the group consisting of a silicone-based compound, an acylated amino acid, and an organic titanate, more preferably includes one or more selected from the group consisting of dimethyl polysiloxane, methyl hydrogen polysiloxane, alkyl alkoxysilane, disodium stearoyl glutamate, sodium lauroyl aspartate, and isopropyl triisostearoyl titanate, further more preferably includes one or more selected from the group consisting of dimethyl polysiloxane, disodium stearoyl glutamate, and isopropyl triisostearoyl titanate, and even more preferably includes disodium stearoyl glutamate.

Hydrophobization treatment may be performed by a normal method.

The amount treated in the hydrophobization treatment is preferably from 0.1 to 15 mass%, more preferably from 1 to 12 mass%, relative to the amount of the powder to be coated, in view of improving the dispersibility into the solvent.

In the case where a color pigment is hydrophobized, the content of the color pigment means the content including the hydrophobization agent.

As the component (G), the color pigments may be used singly or in combinations of two or more thereof. In view of the makeup finish, the content thereof in the whole composition is preferably 0.01 mass% or more, more preferably 0.1 mass% or more, further more preferably 1 mass% or more, even more preferably 5 mass% or more, and preferably 40 mass% or less, more preferably 30 mass% or less, further more preferably 25 mass% or less, and even more preferably 20 mass% or less. Further, the content of the component (G) in the whole composition is preferably from 0.01 to 40 mass%, more preferably from 0.1 to 30 mass%, further more preferably from 1 to 25 mass%, and even more preferably from 5 to 20 mass%.

In the present invention, in view of the viscosity stability and makeup finish, the mass ratio of the component (G) to the component (C), (G)/(C), is preferably 500 or more, more preferably 5 000 or more, further more preferably 8 000 or more, even more preferably 10 000 or more, and preferably 200 000 or less, more preferably 100 000 or less, further more preferably 60 000 or less, and even more preferably 20 000 or less. Also, the mass ratio of the component (G) to the component (C), (G)/(C), is preferably from 500 to 200 000, more preferably from 5 000 to 100 000, further more preferably from 8 000 to 60 000, and even more preferably from 10 000 to 20 000.

In the present invention, in view of the viscosity stability and makeup finish, the content of water in the whole composition is preferably 0.1 mass% or more, more preferably 1 mass% or more, further more preferably 10 mass% or more, and preferably 80 mass% or less, more preferably 70 mass% or less, and further more preferably 50 mass% or less. Further, the content of water in the whole composition is preferably from 0.1 to 80 mass%, more preferably from 1 to 70 mass%, and further more preferably from 10 to 50 mass%.

In addition to the above-described components, the water-in-oil emulsion composition of the present invention may contain components that are commonly used in cosmetics, including an oil component other than those described above, a powder other than those described above, a surfactant other than those described above, a water-soluble polymer, an antioxidant, a fragrance, a preservative, a thickener, a pH adjuster, a blood circulation promoter, a cooling sensation agent, an antiperspirant, a bactericide, a skin activator, a moisturizing agent, and a cooling agent.

The water-in-oil emulsion composition of the present invention may be produced by a conventional method.

The water-in-oil emulsion composition of the present invention may be in a liquid, milky lotion, paste, cream, or gel form.

In view of improving the spreadability when applied, the feel of adhesion to the skin, and the covering ability of the finish, the water-in-oil emulsion composition of the present invention has a viscosity at 30°C of preferably 15 000 mPa·s or more, more preferably 18 000 mPa·s or more, further more preferably 20 000 mPa·s or more, even more preferably 22 000 mPa·s or more, and preferably 200 000 mPa·s or less, more preferably 100 000 mPa·s or less, further more preferably 75 000 mPa·s or less, and even more preferably 50 000 mPa·s or less. Also, the water-in-oil emulsion composition of the present invention has a viscosity at 30°C of preferably from 15 000 to 200 000 mPa·s, more preferably from 18 000 to 100 000 mPa·s, further more preferably from 20 000 to 75 000 mPa·s, and even more preferably from 22 000 to 50 000 mPa·s.

The water-in-oil emulsion composition of the present invention has a relatively high viscosity, and even when the viscosity is lowered by pressure application due to filling or the like, the viscosity is then easily recovered to an original value before pressure application.

The water-in-oil emulsion composition of the present invention is preferably an external preparation composition, more preferably an external preparation composition for the skin.

The water-in-oil emulsion composition of the present invention is preferably a water-in-oil emulsion cosmetic, more preferably a water-in-oil emulsion cosmetic for the skin, further more preferably a UV protective cosmetic and a makeup cosmetic, and even more preferably a makeup cosmetic.

The water-in-oil emulsion composition of the present invention may be used as, for example, a makeup base, a foundation, and a concealer; a makeup cosmetic such as a blusher, an eyeshadow, a mascara, an eyeliner, an eyebrow, an overcoating agent, and a lipstick; a UV protective cosmetic such as a sunscreen lotion and a sunscreen cream; and a skin care cosmetic such as a skin care lotion, a skin care cream, a BB cream, and a beauty serum.

Regarding the embodiment described above, the present invention further discloses the following composition and the like.

<1> A water-in-oil emulsion composition comprising following components (A), (B), (C) and (D):

(A) a cationically modified clay mineral substituted with a quaternary ammonium ion;
(B) a phenyl-modified silicone in liquid form at 2 5°C;
(C) a sorbitan fatty acid ester, wherein a content of the component (C) is from 0.00005 to 0.3 mass%; and
(D) a polyether-modified silicone.

<2> The water-in-oil emulsion composition according to item <1>, wherein the cationically modified clay mineral of the component (A) is preferably obtained by substituting a layered clay mineral with a quaternary ammonium salt-type cationic surfactant, and the cationically modified clay mineral of the component (A) more preferably includes one or more selected from the group consisting of dimethyldistearylammonium hectorite, dimethyldistearylammonium bentonite and benzyldimethylstearylammonium hectorite, further more preferably includes one or more selected from the group consisting of dimethyldistearylammonium hectorite and benzyldimethylstearylammonium hectorite, and even more preferably includes dimethyldistearylammonium hectorite.

<3> The water-in-oil emulsion composition according to item <1> or <2>, wherein the content of the component (A) in the whole composition is preferably 0.01 mass% or more, more preferably 0.1 mass% or more, further more preferably 0.5 mass% or more, even more preferably 0.8 mass% or more, particularly preferably 1.4 mass% or more, and preferably 10 mass% or less, more preferably 5 mass% or less, further more preferably 3 mass% or less, even more preferably 2.5 mass% or less, and particularly preferably 1.8 mass% or less.

<4> The water-in-oil emulsion composition according to any one of items from <1> to <3>, wherein the component (B) has a viscosity at 25°C of preferably 4 000 mPa·s or less, more preferably 600 mPa·s or less, further more preferably 200 mPa·s or less, and even more preferably 50 mPa·s or less.

<5> The water-in-oil emulsion composition according to any one of items from <1> to <4>, wherein the component (B) is one or more selected from the group consisting of phenyl trimethicone and diphenylsiloxy phenyl trimethicone, and the component (B) more preferably includes diphenylsiloxy phenyl trimethicone.

<6> The water-in-oil emulsion composition according to any one of items from <1> to <5>, wherein the content of the component (B) in the whole composition is preferably 1 mass% or more, more preferably 2 mass% or more, further more preferably 3 mass% or more, even more preferably 3.5 mass% or more, particularly preferably 5 mass% or more, and preferably 25 mass% or less, more preferably 20 mass% or less, further more preferably 15 mass% or less, even more preferably 10 mass% or less, and particularly preferably 7 mass% or less.

<7> The water-in-oil emulsion composition according to any one of items from <1> to <6>, wherein the mass ratio of the component (A) to the component (B), (A)/(B), is preferably 0.05 or more, more preferably 0.1 or more, further more preferably 0.12 or more, even more preferably 0.15 or more, more preferably 0.2 or more, even more preferably 0.23 or more, and preferably 1 or less, more preferably 0.5 or less, further more preferably 0.45 or less, even more preferably 0.40 or less, more preferably 0.36 or less, even more preferably 0.3 or less.

<8> The water-in-oil emulsion composition according to any one of items from <1> to <7>, wherein the component (C) has an HLB of preferably 1 or more, more preferably 2 or more, further more preferably 3 or more, and preferably 10 or less, more preferably 7 or less, further more preferably 6 or less.

<9> The water-in-oil emulsion composition according to any one of items from <1> to <8>, wherein the component (C) is preferably one or more selected from the group consisting of sorbitan monoisostearate, sorbitan monooleate and sorbitan sesquistearate, and the component (C) more preferably includes one or more selected from the group consisting of sorbitan monoisostearate and sorbitan sesquistearate, and further more preferably includes sorbitan monoisostearate.

<10> The water-in-oil emulsion composition according to any one of items from <1> to <9>, wherein the content of the component (C) in the whole composition is preferably 0.00007 mass% or more, more preferably 0.00008 mass% or more, further more preferably 0.0001 mass% or more, even more preferably 0.0005 mass% or more, and preferably 0.1 mass% or less, more preferably 0.05 mass% or less, further more preferably 0.005 mass% or less.

<11> The water-in-oil emulsion composition according to any one of items from <1> to <10>, wherein the content of the component (C) in the whole composition is preferably from 0.0001 to 0.3 mass%.

<12> The water-in-oil emulsion composition according to any one of items from <1> to <11>, wherein the mass ratio of the component (B) to the component (C), (B)/(C), is preferably 20 or more, more preferably 60 or more, further more preferably 150 or more, even more preferably 200 or more, even more preferably 2 000 or more, and preferably 80 000 or less, more preferably 60 000 or less, further more preferably 30 000 or less, even more preferably 7 000 or less.

<13> The water-in-oil emulsion composition according to any one of items from <1> to <12>, wherein the component (D) is preferably a dimethylpolysiloxane having a polyoxyethylene group or a polyoxypropylene group, more preferably a dimethylpolysiloxane having at least a polyoxyethylene group.

<14> The water-in-oil emulsion composition according to any one of items from <1> to <13>, wherein the component (D) has an HLB of preferably 1 or more, more preferably 3 or more, and preferably 12 or less, more preferably 10 or less, further more preferably 8 or less, and even more preferably 6 or less.

<15> The water-in-oil emulsion composition according to any one of items from <1> to <14>, wherein the component (D) is preferably one or more selected from the group consisting of cetyl PEG/PPG-10/1 dimethicone, PEG/PPG-19/19 dimethicone, and PEG10-dimethicone, and the component (D) more preferably includes one or more selected from the group consisting of cetyl PEG/PPG-10/1 dimethicone and PEG/PPG-19/19 dimethicone, and further more preferably includes cetyl PEG/PPG-10/1 dimethicone.

<16> The water-in-oil emulsion composition according to any one of items from <1> to <15>, wherein the content of the component (D) in the whole composition is preferably 0.1 mass% or more, more preferably 0.5 mass% or more, further more preferably 1 mass% or more, and preferably 10 mass% or less, more preferably 5 mass% or less, and further more preferably 3 mass% or less.

<17> The water-in-oil emulsion composition according to any one of items from <1> to <16>, further comprising an ester oil (E) in liquid form at 25°C.

<18> The water-in-oil emulsion composition according to item <17>, wherein the component (E) is preferably one or more selected from the group consisting of a monoester, a diester and a triester, and the component (E) more preferably includes one or more selected from the group consisting of a monoester and a diester.

<19> The water-in-oil emulsion composition according to item <17> or <18>, wherein the component (E) is preferably a combination of a monoester and a diester.

<20> The water-in-oil emulsion composition according to any one of items from <17> to <19>, wherein the content of the component (E) in the whole composition is preferably 1 mass% or more, more preferably 2 mass% or more, further more preferably 3 mass% or more, and preferably 20 mass% or less, more preferably 15 mass% or less, further more preferably 10 mass% or less.

<21> The water-in-oil emulsion composition according to any one of items <1> to <20>, further comprising, as a component (F), an oily gelling agent other than the component (A).

<22> The water-in-oil emulsion composition according to item <21>, wherein the component (F) is preferably one or more selected from the group consisting of a crosslinked organopolysiloxane, a dextrin fatty acid ester, and an oily component in solid form at 25°C, and the component (F) is more preferably a crosslinked organopolysiloxane, further more preferably includes one or more selected from the group consisting of a crosslinked dimethylpolysiloxane and a crosslinked alkylpolysiloxane, even more preferably includes one or more selected from the group consisting of a (dimethicone/vinyl dimethicone) crosspolymer, a (dimethicone/phenyl dimethicone) crosspolymer, and a (vinyl dimethicone/(lauryl dimethicone) crosspolymer, and particularly preferably is a (dimethicone/vinyl dimethicone) crosspolymer.

<23> The water-in-oil emulsion composition according to item <21> or <22>, wherein the content of the component (F) in the whole composition is preferably 0.1 mass% or more, more preferably 0.2 mass% or more, further more preferably 0.3 mass% or more, and even more preferably 0.5 mass% or more, and preferably 20 mass% or less, more preferably 10 mass% or less, further more preferably 5 mass% or less, and even more preferably 2 mass% or less.

<24> The water-in-oil emulsion composition according to any one of items from <1> to <23>, further comprising a color pigment (G).

<25> The water-in-oil emulsion composition according to item <24>, wherein the component (G) preferably includes a metal oxide, more preferably includes one or more selected from the group consisting of titanium oxide, zinc oxide, yellow iron oxide, black iron oxide, and red iron oxide, and further more preferably includes one or more selected from the group consisting of titanium oxide, yellow iron oxide, black iron oxide, and red iron oxide.

<26> The water-in-oil emulsion composition according to item <24> or <25>, wherein the component (G) is preferably hydrophobized, and the surface treatment agent preferably includes one or more selected from the group consisting of a silicone-based compound, an acylated amino acid, and an organic titanate, more preferably includes one or more selected from the group consisting of dimethylpolysiloxane, methyl hydrogen polysiloxane, alkyl alkoxysilane, disodium stearoyl glutamate, sodium lauroyl aspartate, and isopropyl triisostearoyl titanate, and further more preferably includes one or more selected from the group consisting of dimethylpolysiloxane, disodium stearoyl glutamate, and isopropyl triisostearoyl titanate, and even more preferably includes disodium stearoyl glutamate.

<27> The water-in-oil emulsion composition according to any one of items from <24> to <26>, wherein the content of the component (G) in the whole composition is preferably 0.01 mass% or more, more preferably 0.1 mass% or more, further more preferably 1 mass% or more, even more preferably 5 mass% or more, and preferably 40 mass% or less, more preferably 30 mass% or less, further more preferably 25 mass% or less, even more preferably 20 mass% or less.

<28> The water-in-oil emulsion composition according to any one of items from <24> to <27>, wherein the mass ratio of the component (G) to the component (C), (G)/(C), is preferably 500 or more, more preferably 5 000 or more, further more preferably 8 000 or more, and even more preferably 10 000 or more, and preferably 200 000 or less, more preferably 100 000 or less, further more preferably 60 000 or less, even more preferably 20 000 or less.

<29> The water-in-oil emulsion composition according to any one of items from <1> to <28>, wherein the content of water in the whole composition is preferably 0.1 mass% or more, more preferably 1 mass% or more, further more preferably 10 mass% or more, and preferably 80 mass% or less, more preferably 70 mass% or less, further more preferably 50 mass% or less.

<30> The water-in-oil emulsion composition according to any one of items from <1> to <29>, having a viscosity at 30°C of preferably 15 000 mPa·s or more, more preferably 18 000 mPa·s or more, further more preferably 20 000 mPa·s or more, even more preferably 22 000 mPa·s or more, and preferably 200 000 mPa·s or less, more preferably 100 000 mPa·s or less, further more preferably 75 000 mPa·s or less, and even more preferably 50 000 mPa·s or less.

<31> The water-in-oil emulsion composition according to any one of items from <1> to <30>,wherein the water-in-oil emulsion composition is preferably an external preparation composition, more preferably an external preparation composition for the skin.

<32> The water-in-oil emulsion composition according to any one of items from <1> to <31>, wherein the water-in-oil emulsion composition is preferably a water-in-oil emulsion cosmetic, more preferably a water-in-oil emulsion cosmetic for the skin, further more preferably a UV protective cosmetic or a makeup cosmetic, and even more preferably a makeup cosmetic.

<33> An external preparation composition for the skin, comprising following components (A), (B), (C) and (D) :

(A) a cationically modified clay mineral substituted with a quaternary ammonium ion, wherein a content of the component (A) is from 0.01 to 10 mass%;
(B) a phenyl-modified silicone in liquid form at 25°C, wherein a content of the component (B) is from 1 to 25 mass%;
(C) a sorbitan fatty acid ester, wherein a content of the component (C) is from 0.00005 to 0.3 mass%; and
(D) a polyether-modified silicone, wherein a content of the component (D) is from 0.1 to 10 mass%.

<34> An external preparation composition for the skin, comprising following components (A), (B), (C) and (D) :

(A) a cationically modified clay mineral substituted with a quaternary ammonium ion, wherein a content of the component (A) is from 0.8 to 2.5 mass%;
(B) a phenyl-modified silicone in liquid form at 25°C, wherein a content of the component (B) is from 3.5 to 10 mass%;
(C) a sorbitan fatty acid ester, wherein a content of the component (C) is from 0.00005 to 0.05 mass%; and
(D) a polyether-modified silicone, wherein a content of the component (D) is from 0.1 to 10 mass%.

<35> An external preparation composition for the skin, comprising following components (A), (B), (C), (D), (E), (F) and (G):

(A) a cationically modified clay mineral substituted with a quaternary ammonium ion, wherein a content of the component (A) is from 0.01 to 10 mass%;
(B) a phenyl-modified silicone in liquid form at 25°C, wherein a content of the component (B) is from 1 to 25 mass%;
(C) a sorbitan fatty acid ester, wherein a content of the component (C) is from 0.00005 to 0.3 mass%;
(D) a polyether-modified silicone, wherein a content of the component (D) is from 0.1 to 10 mass%;
(E) an ester oil in liquid form at 25°C, wherein a content of the component (E) is from 1 to 20 mass%;

(F) an oily gelling agent other than the component (A), wherein a content of the component (F) is from 0.1 to 20 mass%; and
(G) a color pigment, wherein a content of the component (G) is from 0.01 to 40 mass%.

<36> An external preparation composition for the skin, comprising following components (A), (B), (C), (D), (E), (F) and (G):

(A) a cationically modified clay mineral substituted with a quaternary ammonium ion, wherein a content of the component (A) is from 0.8 to 2.5 mass%;
(B) a phenyl-modified silicone in liquid form at 25°C, wherein a content of the component (B) is from 3.5 to 10 mass% or less;
(C) a sorbitan fatty acid ester, wherein a content of the component (C) is from 0.00005 to 0.05 mass%;
(D) a polyether-modified silicone, wherein a content of the component (D) is from 0.1 to 10 mass%;
(E) an ester oil in liquid form at 25°C, wherein a content of the component (E) is from 3 to 10 mass%;
(F) a crosslinked organopolysiloxane, wherein a content of the component (F) is from 0.5 to 2 mass%; and
(G) a color pigment, wherein a content of the component (G) is from 5 to 20 mass%.

<37> Use of the water-in-oil emulsion composition according to any one of items from <1> to <32> as a makeup cosmetic.
<38> Use of the water-in-oil emulsion composition according to any one of items from <1> to <32> for makeup, wherein the water-in-oil emulsion composition is applied to the skin, preferably to the face.
<39> Use of the water-in-oil emulsion composition according to any one of the items from <1> to <32> for production of a water-in-oil emulsion cosmetic.
<40> A water-in-oil emulsion composition produced by blending following components (A), (B), (C) and (D):

(A) a cationically modified clay mineral substituted with a quaternary ammonium ion;
(B) a phenyl-modified silicone in liquid form at 2 5°C;
(C) a sorbitan fatty acid ester, wherein a content of the composition (C) is from 0.00005 to 0.3 mass%; and
(D) a polyether-modified silicone.

Examples

Examples 1 to 12 and Comparative Examples 1 to 2

A water-in-oil emulsion cosmetic (foundation) having a composition shown in Table 1 was produced. The viscosities immediately after production and after one month-storage were measured, and evaluations were also carried out for the viscosity stability when a pressure had been applied by a filling machine, the spreadability when applied, the feel of adhesion to the skin, and the covering ability. The results are also shown in Table 1.
In Comparative Examples 1 and 2, the viscosity stability when a pressure has been applied by a filling machine was not evaluated because of the low viscosities.

(Production method)

A water-in-oil emulsion cosmetic was obtained by adding an aqueous phase component to an oil phase containing the components (A), (B), (C), (D), (E), (F), and (G), and stirring the mixture.

(Evaluation method)

(1) Viscosity measurement

For each of the water-in-oil emulsion cosmetics, the viscosities immediately after production and after one-month storage at 30°C were measured at 30°C with a helical viscometer (TVB-10 type, manufactured by Toki Sangyo Co., Ltd.), with a rotor H-T-C, at a number of rotations of 10, for a measurement time of 60 seconds. These viscosities were obtained without pressure application by the filling machine described in the following (2).
In Table 1, the viscosity values are shown as the following items.

(i): Viscosity immediately after production (30°C) /mPa·s
(ii): Viscosity after 1 month (30°C) /mPa·s

(2) Viscosity stability when a pressure has been applied by a filling machine

A pressure was applied to each of the water-in-oil emulsion cosmetics by a filling machine under the following conditions, then 40 g of each of the water-in-oil emulsion cosmetics was placed in a 50-mL sample bottle, and the viscosity after 1-month storage at 30°C (iii) was measured in the same manner as in (1).

Based on these viscosities, the viscosity stability (viscosity recoverability) when a pressure had been applied by a filling machine was determined by the following formula. The closer to 100%, the smaller the change in viscosity, which is excellent.

Viscosity stability (viscosity recoverability) when

pressure has been applied by filling machine (%)

=(((iii): Viscosity after 1-month storage from pressure application)/((ii) Viscosity after 1 month))×100

· Conditions of pressure application by filling machine: electrically driven piston-type filling machine SBK-1-9L, manufactured by Arahata Food Machine Co., Ltd., cylinder diameter: 40 mm, nozzle inner diameter: 5 mm, filling time: 0.3 (sec)

(3) Spreadability when applied

Three evaluation specialists applied 0.03 g of each of the water-in-oil emulsion cosmetics to an area of $2\times3$ $cm^2$ of the skin on the inner side of a forearm by the fingers, and evaluated the spreadability when applied on the following five-point scale. The results were shown by the total points of the three specialists.

5: Spreading was very good.
4: Spreading was good.
3: Spreading was slightly good.
2: Spreading was not so good.
1: Spreading was poor.

(4) Feel of adhesion to the skin

Three evaluation specialists applied 0.03 g of each of the water-in-oil emulsion cosmetics to an area of $2\times3$ $cm^2$ of the skin on the inner side of a forearm by the fingers. After massage of the skin for 20 seconds at a speed of one reciprocation per second, the feel of adhesion of the cosmetic to the skin was evaluated on the following five-point scale. The results were shown by the total points of the three specialists.

The feel of adhesion to the skin is a feel as if the fingers stick to the skin after the massage of the skin for 20 seconds with an actual feel of close adhesion of the water-in-oil emulsion cosmetic to the skin.

5: A feel of adhesion was clearly given.
4: A feel of adhesion was given.
3: A feel of adhesion was slightly given.
2: A feel of adhesion was rarely given.
1: No feel of adhesion was given.

(5) Covering ability

Three evaluation specialists applied 0.25 g of each of the water-in-oil emulsion cosmetics to the face by the fingers. The covering ability for pores and color unevenness was evaluated on the following five-point scale. The results were shown by the total points of the three specialists.

5: Covering ability was clearly provided.
4: Covering ability was provided.
3: Covering ability was slightly provided.

2: Covering ability was rarely provided.
1: No covering ability was provided.

[Table 1]

| Component (mass%) | | | Example 1 | Example 2 | Example 3 | Example 4 | Example 5 | Example 6 | Example 7 | Example 8 | Example 9 | Example 10 | Example 11 | Example 12 | Comparative Example 1 | Comparative Example 2 |
|---|---|---|---|---|---|---|---|---|---|---|---|---|---|---|---|---|
| (D) | Cetyl PEG/PPG-10/1 dimethicone | *1 | 2 | 2 | 2 | 2 | 2 | 2 | 2 | | 2 | 2 | 2 | 2 | 3 | - |
| | PEG/PPG-19/19 dimethicone (50 mass% isoparaffin dispersion) | *2 | | | | | | | | 4 | | | | | | |
| (C) | Sorbitan isostearate | *3 | 0.001 | 0.001 | 0.001 | 0.001 | 0.001 | 0.0001 | 0.01 | 0.001 | 0.001 | 0.001 | 0.001 | 0.001 | 0.5 | 0.5 |
| | Methyl trimethicone | *4 | 19.839 | 20.039 | 19.339 | 21.839 | 17.839 | 19.8399 | 19.83 | 17.839 | 19.839 | 8.839 | 8.839 | 19.839 | 19.34 | 21.34 |
| (B) | Diphenylsiloxy phenyl trimethicone | *5 | 6 | 6 | 6 | 4 | 8 | 6 | 6 | 6 | 6 | 6 | 6 | 6 | 6 | 6 |
| (E) | Ethylhexyl methoxycinnamate | *6 | 4 | 4 | 4 | 4 | 4 | 4 | 4 | 4 | 7 | 4 | 4 | 4 | 4 | 4 |
| | PG dicaprylate | *7 | 3 | 3 | 3 | 3 | 3 | 3 | 3 | 3 | | 3 | 3 | 3 | 3 | 3 |

EP 4 349 415 A1

| Component (mass%) | | | Example 1 | Example 2 | Example 3 | Example 4 | Example 5 | Example 6 | Example 7 | Example 8 | Example 9 | Example 10 | Example 11 | Example 12 | Comparative Example 1 | Comparative Example 2 |
|---|---|---|---|---|---|---|---|---|---|---|---|---|---|---|---|---|
| (F) | (Dimethicone/vinyl dimethicone) crosspolymer (35 mass% methyl trimethicone dispersion) | *8 | 3 | 3 | 3 | 3 | 3 | 3 | 3 | 3 | 3 | 3 | 3 | | 3 | 3 |
| | crosspolymer (25 mass% dimethylpolysiloxane dispersion) | *9 | | | | | | | | | | | | 4 | | |
| | Dimethiconol | | 1 | 1 | 1 | 1 | 1 | 1 | 1 | 1 | 1 | 1 | 1 | | 1 | 1 |
| | 3 mass% disodium N-stearoyl-L glutamate-treated titanium oxide (80 mass % cyclopentasiloxane dispersion) | *10 | 12.5 | 12.5 | 12.5 | 12.5 | 12.5 | 12.5 | 12.5 | 12.5 | 12.5 | 12.5 | 12.5 | 12.5 | 12.5 | 12.5 |

(continued)

| Component (mass%) | | | Example 1 | Example 2 | Example 3 | Example 4 | Example 5 | Example 6 | Example 7 | Example 8 | Example 9 | Example 10 | Example 11 | Example 12 | Comparative Example 1 | Comparative Example 2 |
|---|---|---|---|---|---|---|---|---|---|---|---|---|---|---|---|---|
| | 3 mass% disodium N-stearoyl-L glutamate-treated red iron oxide (65 mass% cyclopentasiloxane dispersion) | *11 | 0.51 | 0.51 | 0.51 | 0.51 | 0.51 | 0.51 | 0.51 | 0.51 | 0.51 | 0.51 | 0.51 | 0.51 | 0.51 | 0.51 |
| (G) | 3 mass% disodium N-stearoyl-L glutamate-treated yellow iron oxide (70 mass% cyclopentasiloxane dispersion) | *12 | 1.69 | 1.69 | 1.69 | 1.69 | 1.69 | 1.69 | 1.69 | 1.69 | 1.69 | 1.69 | 1.69 | 1.69 | 1.69 | 1.69 |
| | 3 mass% disodium N-stearoyl-L glutamate-treated black iron oxide (75 mass% cyclopentasiloxane dispersion) | *13 | 0.21 | 0.21 | 0.21 | 0.21 | 0.21 | 0.21 | 0.21 | 0.21 | 0.21 | 0.21 | 0.21 | 0.21 | 0.21 | 0.21 |

(continued)

| (A) Component (mass%) | | Example 1 | Example 2 | Example 3 | Example 4 | Example 5 | Example 6 | Example 7 | Example 8 | Example 9 | Example 10 | Example 11 | Example 12 | Comparative Example 1 | Comparative Example 2 |
|---|---|---|---|---|---|---|---|---|---|---|---|---|---|---|---|
| Dimethyldistearylammonium hectorite | *14 | 1.5 | 1.3 | 2 | 1.5 | 1.5 | 1.5 | 1.5 | 1.5 | 1.5 | | | 1.5 | 1.5 | 1.5 |
| Benzyl dimethyl stearyl ammonium hectorite (12.5 mass% (triglyceryl (caprylate/caprate)/propylene carbonate mixture) dispersion) | *15 | | | | | | | | | | 12.5 | | | | |
| Benzyl dimethyl stearyl ammonium hectorite (12.5 mass% (alkyl benzoate (from C12 to C15)/propylene carbonate mixture) dispersion) | *16 | | | | | | | | | | | 12.5 | | | |

(continued)

| Component (mass%) | | Example 1 | Example 2 | Example 3 | Example 4 | Example 5 | Example 6 | Example 7 | Example 8 | Example 9 | Example 10 | Example 11 | Example 12 | Comparative Example 1 | Comparative Example 2 |
|---|---|---|---|---|---|---|---|---|---|---|---|---|---|---|---|
| | Ethanol | 1 | 1 | 1 | 1 | 1 | 1 | 1 | 1 | 1 | 1 | 1 | 1 | 1 | 1 |
| | 1,3-butylene glycol | 6 | 6 | 6 | 6 | 6 | 6 | 6 | 6 | 6 | 6 | 6 | 6 | 6 | 6 |
| | Phenoxyethanol | 0.5 | 0.5 | 0.5 | 0.5 | 0.5 | 0.5 | 0.5 | 0.5 | 0.5 | 0.5 | 0.5 | 0.5 | 0.5 | 0.5 |
| | Sodium chloride | 1.25 | 1.25 | 1.25 | 1.25 | 1.25 | 1.25 | 1.25 | 1.25 | 1.25 | 1.25 | 1.25 | 1.25 | 1.25 | 1.25 |
| | purified water | 36 | 36 | 36 | 36 | 36 | 36 | 36 | 36 | 36 | 36 | 36 | 36 | 36 | 36 |
| Total | | 100.00 | 100.00 | 100.00 | 100.00 | 100.00 | 100.00 | 100.00 | 100.00 | 100.00 | 100.00 | 100.00 | 100.00 | 100.00 | 100.00 |
| Total amount of (A) | | 1.5 | 1.3 | 2 | 1.5 | 1.5 | 1.5 | 1.5 | 1.5 | 1.5 | 1.56 | 1.56 | 1.5 | 1.5 | 1.5 |
| Total amount of (B) | | 6 | 6 | 6 | 4 | 8 | 6 | 6 | 6 | 6 | 6 | 6 | 6 | 6 | 6 |
| Total amount of (C) | | 0.001 | 0.001 | 0.001 | 0.001 | 0.001 | 0.0001 | 0.01 | 0.001 | 0.001 | 0.001 | 0.001 | 0.001 | 0.5 | 0.5 |
| Total amount of (D) | | 2 | 2 | 2 | 2 | 2 | 2 | 2 | 2 | 2 | 2 | 2 | 2 | 2 | |
| Total amount of (E) | | 7 | 7 | 7 | 7 | 7 | 7 | 7 | 7 | 7 | 7 | 7 | 7 | 7 | 7 |
| Total amount of (F) | | 1.05 | 1.05 | 1.05 | 1.05 | 1.05 | 1.05 | 1.05 | 1.05 | 1.05 | 1.05 | 1.05 | 1 | 1.05 | 1.05 |
| Total amount of (G) | | 11.672 | 11.672 | 11.672 | 11.672 | 11.672 | 11.672 | 11.672 | 11.672 | 11.672 | 11.672 | 11.672 | 11.672 | 11.672 | 11.672 |
| (A)/(B) | | 0.25 | 0.22 | 0.33 | 0.38 | 0.19 | 0.25 | 0.25 | 0.25 | 0.25 | 0.26 | 0.26 | 0.25 | 0.25 | 0.25 |
| (B)/(C) | | 6000 | 6000 | 6000 | 4000 | 6000 | 60000 | 600 | 6000 | 6000 | 6000 | 6000 | 6000 | 12 | 12 |
| (G)/(C) | | 11672 | 11672 | 11672 | 11672 | 11672 | 116720 | 1167 | 11672 | 11672 | 11672 | 11672 | 11672 | 23 | 23 |
| (i): Viscosity immediately after production (30°C) /mPa·s | | 30,700 | 29,900 | 36,300 | 28,500 | 32,700 | 27,000 | 27,000 | 31,700 | 24,000 | 85,000 | 63,700 | 30,700 | 16,600 | 7,600 |
| (ii): Viscosity after 1 month (30°C)/mPa·s | | 34,000 | 28,600 | 41,600 | 34,800 | 35,700 | 29,900 | 29,900 | 42,900 | 26,100 | 57,100 | 42,000 | 32,500 | 5 000 or less | 3,200 |

| Component (mass%) | | Example 1 | Example 2 | Example 3 | Example 4 | Example 5 | Example 6 | Example 7 | Example 8 | Example 9 | Example 10 | Example 11 | Example 12 | Compara-tive Example 1 | Compara-tive Example 2 |
|---|---|---|---|---|---|---|---|---|---|---|---|---|---|---|---|
| Viscosity stability when pressure has been applied by filling machine ((iii: Viscosity after 1-month storage from pressure application)/((ii) Viscosity after 1 month))×100 | | 71.0% | 81.6% | 85.5% | 75.0% | 86.1% | 83.0% | 83.0% | 93.6% | 77.0% | 91.0% | 111.6% | 84.2% | - | - |
| Spreadability when applied | | 15 | 14 | 12 | 14 | 15 | 15 | 15 | 13 | 14 | 11 | 13 | 15 | 3 | 6 |
| Feel of adhesion to skin | | 15 | 13 | 11 | 12 | 14 | 11 | 11 | 12 | 12 | 12 | 12 | 12 | 3 | 5 |
| Covering ability | | 15 | 15 | 15 | 14 | 14 | 13 | 13 | 15 | 12 | 10 | 10 | 12 | 3 | 6 |

EP 4 349 415 A1

The trade names of the components which were used in Examples are as follows.

*1: Cetyl PEG/PPG-10/1 dimethicone; ABIL EM 90, manufactured by Evonik Operations GmbH

*2: PEG/PPG-19/19 dimethicone (50 mass% isoparaffin dispersion); DOWSIL BY 25-337, manufactured by Dow Toray Co., Ltd.

*3: Sorbitan isostearate; Span 120-LQ-(RB), manufactured by Croda Europe Ltd.

*4: Methyl trimethicone; TMF-1.5(-G), manufactured by Shin-Etsu Chemical Co., Ltd.

*5: Diphenylsiloxy phenyl trimethicone; Silicone KF-56A, manufactured by Shin-Etsu Chemical Co., Ltd.

*6: Ethylhexyl methoxycinnamate; Uvinul MC80, manufactured by BASF SE

* 7: PG dicaprylate; NIKKOL SEFSOL-228, manufactured by Nippon Surfactant Industries Co., Ltd.

*8: (Dimethicone/vinyl dimethicone) crosspolymer (35 mass% methyl trimethicone dispersion), TOREFIL (35%) C/TMF (polymer: 35 mass%, methyl trimethicone: 65 mass%), manufactured by Daito Kasei Kogyo Co., Ltd.

*9: (Dimethicone/vinyl dimethicone) crosspolymer (25 mass% dimethylpolysiloxane dispersion); Silicone KSG-16 (polymer: 25 mass%, dimethylpolysiloxane: 75 mass%), manufactured by Shin-Etsu Chemical Co., Ltd.

*10: Disodium N-stearoyl-L glutamate, 3 mass% treated titanium oxide (80 mass% cyclopentasiloxane dispersion); SA/NAI-TR-10/D5 (80%) MCD (color pigment: 80 mass%, cyclopentasiloxane: 20 mass%), manufactured by Miyoshi Kasei Co., Ltd.

*11: Disodium N-stearoyl-L glutamate, 3 mass% treated red iron oxide (65 mass% cyclopentasiloxane dispersion); SA/NAI-R-10/D5 (65%) MCD (color pigment: 65 mass%, cyclopentasiloxane: 35 mass%), manufactured by Miyoshi Kasei Co., Ltd.

*12: Disodium N-stearoyl-L glutamate, 3 mass% treated yellow iron oxide (70 mass% cyclopentasiloxane dispersion); SA/NAI-Y-10/D5 (70%) MCD (color pigment: 70 mass%, cyclopentasiloxane: 30 mass%), manufactured by Miyoshi Kasei Co., Ltd.

*13: Disodium N-stearoyl-L glutamate, 3 mass% treated black iron oxide (75 mass% cyclopentasiloxane dispersion); SA/NAI-B-10/D5 (75%) MCD (color pigment: 75 mass%, cyclopentasiloxane: 25 mass%), manufactured by Miyoshi Kasei Co., Ltd.

*14: Dimethyldistearylammonium hectorite; Benton 38VCG, manufactured by Elementis Specialties, Inc. (US)

*15: Benzyl dimethyl stearyl ammonium hectorite (12.5 mass% (glyceryl tri(caprylate/caprate)/propylene carbonate mixture) dispersion); BENTONE GEL GTCC V (clay mineral: 12.5 mass%), manufactured by Elementis Specialties, Inc. (US)

*16: Benzyl dimethyl stearyl ammonium hectorite (12.5 mass% (alkyl benzoate (from C12 to C15)/propylene carbonate mixture) dispersion); BENTONE GEL TN V (clay mineral: 12.5 mass%) manufactured by Elementis Specialties, Inc. (US)

Formulation Examples from 1 to 9

Water-in-oil emulsion cosmetics having compositions shown in Tables from 2 to 4 were produced in the same manner as in Examples from 1 to 12.

All of the resulting water-in-oil emulsion cosmetics had good viscosity stability to easily recover the viscosity thereof even when the viscosity was lowered by the pressure application to the cosmetic by the filling machine, and were excellent in the spreadability when applied, the feel of adhesion to the skin and the covering ability of the finish.

[Table 2]

| Component (mass%) | | | Formulation Example 1 | Formulation Example 2 | Formulation Example 3 | Formulation Example 4 | Formulation Example 5 | Formulation Example 6 |
|---|---|---|---|---|---|---|---|---|
| (D) | Cetyl PEG/PPG-10/1 dimethicone | *1 | 2 | 2 | | 2 | 2 | |
| | PEG/PPG-19/19 dimethicone (50 mass% cyclopentasiloxane dispersion) | *17 | | | | | | 4 |
| | PEG10-dimethicone | *18 | | | 2 | | | |
| (C) | Sorbitan isostearate | *3 | 0.001 | 0.001 | 0.001 | 0.0002 | 0.0002 | 0.0002 |
| | Methyl trimethicone | *4 | 19.839 | 19.839 | 19.839 | 19.8398 | 15.8398 | 18.8398 |
| (B) | Diphenylsiloxy phenyl trimethicone | *19 | | 3 | | | | |
| | Diphenylsiloxy phenyl trimethicone | *5 | | | 6 | 3 | 3 | 3 |
| | Phenyl trimethicone | *20 | 6 | 3 | | 3 | 3 | 3 |
| (E) | Ethylhexyl methoxycinnamate | *6 | 4 | 4 | 4 | 4 | 4 | 4 |
| | PG dicaprylate | *7 | 3 | 3 | 3 | 3 | 3 | 3 |
| (F) | (Dimethicone/vinyl dimethicone) crosspolymer (35 mass% methyl trimethicone dispersion) | *8 | 3 | 3 | 3 | 3 | 3 | 3 |
| | Dimethiconol | | 1 | 1 | 1 | 1 | 1 | |
| | Ethanol | | | | | | 4 | |
| (G) | 3 mass% disodium N-stearoyl-L glutamate-treated titanium oxide (80 mass% cyclopentasiloxane dispersion) | *10 | 12.5 | 12.5 | 12.5 | 12.5 | 12.5 | 12.5 |
| | 3 mass% disodium N-stearoyl-L glutamate-treated red iron oxide (65 mass% cyclopentasiloxane dispersion) | *11 | 0.51 | 0.51 | 0.51 | 0.51 | 0.51 | 0.51 |
| | 3 mass% disodium N-stearoyl-L glutamate-treated yellow iron oxide (70 mass% cyclopentasiloxane dispersion) | *12 | 1.69 | 1.69 | 1.69 | 1.69 | 1.69 | 1.69 |
| | 3 mass% disodium N-stearoyl-L glutamate-treated black iron oxide (75 mass% cyclopentasiloxane dispersion) | *13 | 0.21 | 0.21 | 0.21 | 0.21 | 0.21 | 0.21 |
| (A) | Dimethyldistearylammonium hectorite | *14 | 1.5 | 1.5 | 1.5 | 1.5 | 1.5 | 1.5 |

| Component (mass%) | | Formulation Example 1 | Formulation Example 2 | Formulation Example 3 | Formulation Example 4 | Formulation Example 5 | Formulation Example 6 |
|---|---|---|---|---|---|---|---|
| Ethanol | | 1 | 1 | 1 | 1 | 1 | 1 |
| 1,3-butylene glycol | | 6 | 6 | 6 | 6 | 6 | 6 |
| Phenoxy ethanol | | 0.5 | 0.5 | 0.5 | 0.5 | 0.5 | 0.5 |
| Sodium chloride | | 1.25 | 1.25 | 1.25 | 1.25 | 1.25 | 1.25 |
| Purified water | | 36 | 36 | 36 | 36 | 36 | 36 |
| Total | | 100.00 | 100.00 | 100.00 | 100.00 | 100.00 | 100.00 |
| Total amount of (A) | | 1.5 | 1.5 | 1.5 | 1.5 | 1.5 | 1.5 |
| Total amount of (B) | | 6 | 6 | 6 | 6 | 6 | 6 |
| Total amount of (C) | | 0.001 | 0.001 | 0.001 | 0.0002 | 0.0002 | 0.0002 |
| Total amount of (D) | | 2 | 2 | 2 | 2 | 2 | 2 |
| Total amount of (E) | | 7 | 7 | 7 | 7 | 7 | 7 |
| Total amount of (F) | | 1.05 | 1.05 | 1.05 | 1.05 | 1.05 | 1.05 |
| Total amount of (G) | | 11.672 | 11.672 | 11.672 | 11.672 | 11.672 | 11.672 |
| (A)/(B) | | 0.25 | 0.25 | 0.25 | 0.25 | 0.25 | 0.25 |
| (B)/(C) | | 6000 | 6000 | 6000 | 30000 | 30000 | 30000 |
| (G)/(C) | | 11672 | 11672 | 11672 | 58360 | 58360 | 58360 |

*17: PEG/PPG-19/19 dimethicone (50 mass% cyclopentasiloxane dispersion); DOWSIL BY 11-030, manufactured by Dow Silicone Corporation *18: PEG10-dimethicone; KF-6017, manufactured by Shin-Etsu Chemical Co., Ltd.

*19: Diphenylsiloxy phenyl trimethicone; DOWSIL FZ-209, manufactured by Dow Toray Co., Ltd.

*20: Phenyl trimethicone; DOWSIL SH 556 FLUID, manufactured by Dow Toray Co., Ltd.

[Table 3]

| | | Component (mass%) | | Formulation Example 7 | Formulation Example 8 |
|---|---|---|---|---|---|
| (D) | | Cetyl PEG/PPG-10/1 dimethicone | *1 | 2 | 2 |
| (C) | | Sorbitan isostearate | *3 | 0.001 | 0.001 |
| | | Methyl trimethicone | *4 | 7 | 11.6 |
| (B) | | Diphenylsiloxy phenyl trimethicone | *5 | 3 | 3 |
| | | Phenyl trimethicone | *20 | 3 | 3 |
| (E) | | Ethylhexyl methoxycinnamate | *6 | 7 | 4 |
| | | Cetyl ethylhexanoate | *21 | | 3 |
| (F) | | (Dimethicone/vinyl dimethicone) crosspolymer (25 mass% dimethicone dispersion) | *9 | 3 | 3 |
| | | Dimethiconol | | 1 | 1 |
| | | Ethanol | | 2.8 | 4 |
| | | Poly (N-propionyl ethyl imine)-modified silicone | *22 | 1.2 | |
| | | 2-[4-(Diethylamino)-2-hydroxybenzoyl]benzoic acid hexyl ester | *23 | 1 | |
| | | Dimethicodiethylbenzalmalonate | *24 | 2 | |
| | | 2,4-bis-[{4-(2-ethylhexyloxy)-2-hydroxy}-phenyl]-6-(4-methoxy phenyl)-1,3,5-triazine | *25 | 1 | |
| (G) | | 3 mass% disodium N-stearoyl-L glutamate-treated titanium oxide (80 mass% cyclopentasiloxane dispersion) | *10 | 2 | 10 |
| | | 3 mass% disodium N-stearoyl-L glutamate-treated red iron oxide (65 mass% cyclopentasiloxane dispersion) | *11 | 0.1 | 0.5 |
| | | 3 mass% disodium N-stearoyl-L glutamate-treated yellow iron oxide (70 mass% cyclopentasiloxane dispersion) | *12 | 1 | 2 |
| | | 3 mass% disodium N-stearoyl-L glutamate-treated black iron oxide (75 mass% cyclopentasiloxane dispersion) | *13 | 0.05 | 0.1 |
| | | Fine particles of titanium oxide (50 mass% cyclopentasiloxane dispersion) | *26 | 5 | 4 |
| | | Fine particles of zinc oxide (45 mass% cyclopentasiloxane dispersion) | *27 | 12 | |
| | | Dimethylpolysiloxane-treated silicic anhydride | *28 | | 1 |
| | | Dimethylpolysiloxane-treated zinc oxide | *29 | 1 | |
| (A) | | Dimethyldistearylammonium hectorite | *14 | 2 | 1.5 |
| | | Ethanol | | 1 | 1 |
| | | 1,3-butylene glycol | | 6 | 2 |
| | | Glycerol | | | 2 |
| | | Dipropylene glycol | | | 2 |
| | | Sodium hyaluronate | | | 0.001 |
| | | Phenoxy ethanol | | 0.5 | 0.5 |
| | | Sodium chloride | | 1.25 | 1.25 |
| | | Purified water | | 34.099 | 37.548 |

(continued)

| Component (mass%) | Formulation Example 7 | Formulation Example 8 |
|---|---|---|
| Total | 100 | 100 |
| Total amount of (A) | 2 | 1.5 |
| Total amount of (B) | 6 | 6 |
| Total amount of (C) | 0.001 | 0.001 |
| Total amount of (D) | 2 | 2 |
| Total amount of (E) | 7 | 7 |
| Total amount of (F) | 0.75 | 0.75 |
| Total amount of (G) | 11.3025 | 12.8 |
| (A)/(B) | 0.33 | 0.25 |
| (B)/(C) | 6000 | 6000 |
| (G)/(C) | 11302.5 | 12800 |

*21: Cetyl ethylhexanoate; Saracos 816 (L), manufactured by Nisshin Oillio Group *22: Poly(N-propionyl ethyl imine)-modified silicone; JP-A-2016-222599, compound described in Synthesis Example 3 *23: 2-[4-(Diethylamino)-2-hydroxybenzoyl]benzoic acid hexyl ester; Uvinul A PLUS GRANULAR, manufactured by BASF SE *24: Dimethicodiethylbenzalmalonate; Parsol SLX, manufactured by DSM Nutritional Products Ltd.

*25: 2,4-bis-[{4-(2-ethylhexyloxy)-2-hydroxy}-phenyl]-6-(4-methoxy phenyl)-1,3,5-triazine; TINOSORB S, manufactured by BASF SE *26: Fine particles of titanium oxide (50 mass% cyclopentasiloxane dispersion); SA-UT-A40/D5, manufactured by Miyoshi Kasei Co., Ltd.

*27: Fine particles of zinc oxide (45 mass% cyclopentasiloxane dispersion); OSMZ300/45% slurry, manufactured by Tayka Corporation *28: Dimethylpolysiloxane-treated silicic anhydride; MIYOFEEL SA-SB-N1, manufactured by Miyoshi Kasei Co., Ltd.

*29: Dimethylpolysiloxane-treated zinc oxide; MZY-505M, manufactured by Tayka Corporation

[Table 4]

| Component (mass%) | | | Formulation Example 9 |
|---|---|---|---|
| (D) | Cetyl PEG/PPG-10/1 dimethicone | *1 | 2 |
| (C) | Sorbitan isostearate | *3 | 0.001 |
| (B) | Diphenylsiloxy phenyl trimethicone | *5 | 3 |
| | Phenyl trimethicone | *20 | 3 |
| (E) | Ethylhexyl methoxycinnamate | *6 | 4 |
| | Cetyl ethylhexanoate | *21 | 3 |
| | Dimethiconol | | 1 |
| | Ethanol | | 4 |
| | Hydrogenated polyisobutene | *30 | 7 |
| | Hydrogenated polyisobutene | *31 | 6.6 |

(continued)

| Component (mass%) | | | Formulation Example 9 |
|---|---|---|---|
| (G) | Fine particles of titanium oxide (50 mass% cyclopentasiloxane dispersion) | *26 | 4 |
| | Dimethylpolysiloxane-treated silicic anhydride | *28 | 1 |
| | ASI-treated titanium oxide | *32 | 10 |
| | ASI-treated red iron oxide | *33 | 0.5 |
| | ASI-treated yellow iron oxide | *34 | 2 |
| | ASI-treated black iron oxide | *35 | 0.1 |
| (A) | Dimethyldistearylammonium hectorite | *14 | 2.5 |
| | Ethanol | | 1 |
| | 1,3-butylene glycol | | 2 |
| | Glycerol | | 2 |
| | Dipropylene glycol | | 2 |
| | Sodium hyaluronate | | 0.001 |
| | Phenoxy ethanol | | 0.5 |
| | Sodium chloride | | 1.25 |
| | Purified water | | 37.548 |
| Total | | | 100 |
| Total amount of (A) | | | 2.5 |
| Total amount of (B) | | | 6 |
| Total amount of (C) | | | 0.001 |
| Total amount of (D) | | | 2 |
| Total amount of (E) | | | 7 |
| Total amount of (G) | | | 15.6 |
| (A)/(B) | | | 0.42 |
| (B)/(C) | | | 6000 |
| (G)/(C) | | | 15600 |

*30: Hydrogenated polyisobutene; Chloratum LES-LQ, manufactured by Croda Japan *31: Hydrogenated polyisobutene; Pearlreem 4, manufactured by NOF Corporation *32: ASI-treated titanium oxide; BCASI TIO2 MP-701 (98 mass% of titanium oxide coated with 1.5 mass% of isopropyl triisostearoyl titanate, 0.42 mass% of sodium lauroyl aspartate, and 0.08 mass% of zinc chloride), manufactured by Daito Kasei Kogyo Co., Ltd.

*33: ASI-treated red iron oxide; ASI RED R-516P (98 mass% of red iron oxide surface-treated in the same manner as in *32), manufactured by Daito Kasei Co., Ltd.

*34: ASI-treated yellow iron oxide; ASI YELLOW LL-100P (98 mass% of yellow iron oxide surface-treated in the same manner as in *32), manufactured by Daito Kasei Kogyo Co., Ltd.

*35: ASI-treated black iron oxide; ASI BLACK BL-100P (98 mass% of black iron oxide surface treated in the same manner as in *2), manufactured by Daito Kasei Kogyo Co., Ltd.

Examples 13 and 14

A water-in-oil emulsion cosmetic (foundation) having a composition shown in Table 5 was produced in the same manner as in Examples 1 to 12. The viscosities immediately after production and after one month-storage were measured, and evaluations were also carried out for the viscosity stability when a pressure had been applied by a filling machine, the spreadability when applied, the feel of adhesion to the skin, and the covering ability. The results are also shown in Table 5.

[Table 5]

| Component (mass%) | | | | Example 13 | Example 14 |
|---|---|---|---|---|---|
| (D) | | Cetyl PEG/PPG-10/1 dimethicone | *1 | 2 | 2 |
| (C) | | Sorbitan isostearate | *3 | 0.07 | 0.2 |
| | | Methyl trimethicone | *4 | 19.769 | 19.639 |
| (B) | | Diphenylsiloxy phenyl trimethicone | *5 | 6 | 6 |
| (E) | | Ethylhexyl methoxycinnamate | *6 | 4 | 4 |
| | | PG dicaprylate | *7 | 3 | 3 |
| (F) | | (Dimethicone/vinyl dimethicone) crosspolymer (35 mass% methyl trimethicone dispersion) | *8 | 3 | 3 |
| | | Dimethiconol | | 1 | 1 |
| (G) | | 3 mass% disodium N-stearoyl-L glutamate-treated titanium oxide (80 mass% cyclopentasiloxane dispersion) | *10 | 12.5 | 12.5 |
| | | 3 mass% disodium N-stearoyl-L glutamate-treated red iron oxide (65 mass% cyclopentasiloxane dispersion) | *11 | 0.51 | 0.51 |
| | | 3 mass% disodium N-stearoyl-L glutamate-treated yellow iron oxide (70 mass% cyclopentasiloxane dispersion) | *12 | 1.69 | 1.69 |
| | | 3 mass% disodium N-stearoyl-L glutamate-treated black iron oxide (75 mass% cyclopentasiloxane dispersion) | *13 | 0.21 | 0.21 |
| (A) | | Dimethyldistearylammonium hectorite | *14 | 1.5 | 1.5 |
| | | Ethanol | | 1 | 1 |
| | | 1,3-butylene glycol | | 6 | 6 |
| | | Phenoxy ethanol | | 0.5 | 0.5 |
| | | Sodium chloride | | 1.25 | 1.25 |
| | | Purified water | | 36 | 36 |
| Total | | | | 100.00 | 100.00 |
| Total amount of (A) | | | | 1.5 | 1.5 |
| Total amount of (B) | | | | 6 | 6 |
| Total amount of (C) | | | | 0.07 | 0.2 |
| Total amount of (D) | | | | 2 | 2 |
| Total amount of (E) | | | | 7 | 7 |
| Total amount of (F) | | | | 1.05 | 1.05 |
| Total amount of (G) | | | | 11.672 | 11.672 |
| (A)/(B) | | | | 0.25 | 0.25 |
| (B)/(C) | | | | 85.7 | 30 |
| (G)/(C) | | | | 166.7 | 58.36 |
| (i): Viscosity immediately after production(30°C)/mPa·s | | | | 26,500 | 20,800 |

(continued)

| Component (mass%) | | Example 13 | Example 14 |
|---|---|---|---|
| (ii): Viscosity after 1 month (30°C)/mPa·s | | 28,700 | 23,800 |
| Viscosity stability when pressure has been applied by filling machine ((iii): Viscosity after 1-month storage from pressure application)/((ii) Viscosity after 1 month))×100 | | 76.0% | 70.0% |
| Spreadability when applied | | 15 | 15 |
| Feel of adhesion to skin | | 14 | 12 |
| Covering ability | 13 | 13 | |

**Claims**

1.  A water-in-oil emulsion composition comprising components (A), (B), (C) and (D):

    (A) a cationically modified clay mineral substituted with a quaternary ammonium ion;
    (B) a phenyl-modified silicone in liquid form at 25°C;
    (C) a sorbitan fatty acid ester, wherein a content of the component (C) is from 0.00005 to 0.3 mass%; and
    (D) a polyether-modified silicone.

2.  The water-in-oil emulsion composition according to claim 1, wherein a mass ratio of the component (B) to the component (C), (B)/(C), is from 20 to 80 000.

3.  The water-in-oil emulsion composition according to claim 1 or 2, wherein a content of the component (A) is from 0.01 to 10 mass%.

4.  The water-in-oil emulsion composition according to any one of claims 1 to 3, wherein a content of the component (B) is from 1 to 25 mass%.

5.  The water-in-oil emulsion composition according to any one of claims 1 to 4, wherein a content of the component (D) is from 0.1 to 10 mass%.

6.  The water-in-oil emulsion composition according to any one of claims 1 to 5, wherein a content of the component (C) is from 0.0001 to 0.3 mass%.

7.  The water-in-oil emulsion composition according to any one of claims 1 to 6, wherein a mass ratio of the component (A) to the component (B), (A)/(B), is from 0.05 to 1.

8.  The water-in-oil emulsion composition according to any one of claims 1 to 7, further comprising an ester oil (E) in liquid form at 25°C.

9.  The water-in-oil emulsion composition according to any one of claims 1 to 8, further comprising an oily gelling agent.

10. The water-in-oil emulsion composition according to any one of claims 1 to 9, further comprising a color pigment.

11. The water-in-oil emulsion composition according to any one of claims 1 to 10, wherein the water-in-oil emulsion composition is a water-in-oil emulsion cosmetic for the skin.

12. Use of the water-in-oil emulsion composition according to any one of claims 1 to 11 as a makeup cosmetic.

13. Use of the water-in-oil emulsion composition according to any one of claims 1 to 11 for makeup, wherein the water-in-oil emulsion composition is applied to the skin, preferably to the face.

14. Use of the water-in-oil emulsion composition according to any one of claims 1 to 11 for production of a water-in-oil emulsion cosmetic.

15. A water-in-oil emulsion composition produced by blending components (A), (B), (C) and (D):

(A) a cationically modified clay mineral substituted with a quaternary ammonium ion;
(B) a phenyl-modified silicone in liquid form at 25°C;
(C) a sorbitan fatty acid ester, wherein a content of the component (C) is from 0.00005 to 0.3 mass%; and
(D) a polyether-modified silicone.

## INTERNATIONAL SEARCH REPORT

| International application No. |
| --- |
| **PCT/JP2022/021368** |

### A. CLASSIFICATION OF SUBJECT MATTER

*A61Q 1/00*(2006.01)i; *A61K 8/06*(2006.01)i; *A61K 8/19*(2006.01)i; *A61K 8/37*(2006.01)i; *A61K 8/891*(2006.01)i; *A61K 8/894*(2006.01)i
FI: A61K8/06; A61K8/19; A61K8/891; A61K8/37; A61K8/894; A61Q1/00

According to International Patent Classification (IPC) or to both national classification and IPC

### B. FIELDS SEARCHED

Minimum documentation searched (classification system followed by classification symbols)

A61Q1/00; A61K8/06; A61K8/19; A61K8/37; A61K8/891; A61K8/894

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

Mintel GNPD

### C. DOCUMENTS CONSIDERED TO BE RELEVANT

| Category* | Citation of document, with indication, where appropriate, of the relevant passages | Relevant to claim No. |
| --- | --- | --- |
| X | JP 2010-111634 A (SHISEIDO CO LTD) 20 May 2010 (2010-05-20) | 1-8,10-15 |
| | claims, paragraphs [0008], [0018], [0051], [0071], example 6 | |
| Y | | 9 |
| Y | JP 2008-201701 A (TOKIWA CORP) 04 September 2008 (2008-09-04) | 9 |
| | claims, paragraphs [0007], [0012]-[0013] | |
| A | | 1-8, 10-15 |
| X | JP 2016-199482 A (ISEHAN KK) 01 December 2016 (2016-12-01) | 1-18, 10-15 |
| | claims, paragraphs [0006], [0038], [0040], [0046], [0050], table 1, example 2 | |
| Y | | 9 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **12 July 2022** | **26 July 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

International application No.

**PCT/JP2022/021368**

| Patent document cited in search report | | | Publication date (day/month/year) | Patent family member(s) | Publication date (day/month/year) |
|---|---|---|---|---|---|
| JP | 2010-111634 | A | 20 May 2010 | (Family: none) | |
| JP | 2008-201701 | A | 04 September 2008 | (Family: none) | |
| JP | 2016-199482 | A | 01 December 2016 | (Family: none) | |

Form PCT/ISA/210 (patent family annex) (January 2015)

**EP 4 349 415 A1**

### Patent documents cited in the description

- JP 2018070516 A
- JP 2016222599 A